# EUROPEAN PATENT SPECIFICATION

(11) **EP 2 205 152 B1**
(45) Date of publication and mention of the grant of the patent: **23.11.2011**
(21) Application number: 08804095.1
(22) Date of filing: 12.09.2008
(51) Int. Cl.: A61B 5/151

(54) **DISPOSABLE DEVICE FOR ANALYZING BODY FLUID**
EINWEGVORRICHTUNG ZUR ANALYSE EINER KÖRPERFLÜSSIGKEIT
DISPOSITIF JETABLE DESTINÉ À L'ANALYSE DE FLUIDE CORPOREL

(30) Priority: 17.09.2007 US 972996 P
(43) Date of publication of application: 14.07.2010
(73) Proprietor: F. Hoffmann-La Roche AG, 4070 Basel (CH); Roche Diagnostics GmbH, 68305 Mannheim (DE)
(72) Inventor: FREY, Stephan-Michael, 64347 Griesheim (DE); LIST, Hans, 64754 Hesseneck-Kailbach (DE); BRAENDLE, Hansjoerg, CH-9244 Niederuzwil (CH); HOERAUF, Christian, 68723 Oftersheim (DE); ROE, Steven N., San Mateo, California 94403 (US)
(74) Representative: Pfiz, Thomas
(86) International application number: PCT/EP2008/062130
(87) International publication number: WO 2009/037192

(56) References cited:
- EP-A- 1 508 304
- EP-A- 1 759 633
- EP-A- 1 839 576
- DE-A1- 10 345 663
- US-A1- 2007 016 103

## Description

The invention concerns a disposable device for analyzing body fluid especially for blood sugar tests comprising a container which can be inserted into a measuring apparatus, a sampling member provided in the container to pierce skin of a body part and sample body fluid, and a test member for receiving body fluid obtained by the skin-piercing.

Such methods and withdrawal devices for small amounts of body fluids are used above all by diabetics for the self-monitoring of blood sugar which should be carried out several times daily. Recent concepts envisage a microneedle including a test element as a disposable in a hand-held device to generate a skin puncture, to remove a small amount of blood therefrom utilizing capillary forces and to analyse this blood sample. Generally, in such devices the lancing member and the test element are monolithically integrated and and must be handled as combined units, where the lancing and blood transfer require separate actuation. An integrated sampling and measuring device is known from EP A-1759633. Fluid transfer from the sampling member to the test member is initiated by displacing a membrane.

On this basis the object of the invention is to improve the devices Known in the prior art and to provide a simple and low cost concept for an integrated sampling and measuring device and process.

The combination of features stated in the independent patent claims are proposed to achieve this object. Advantageous embodiments and further developments are derived from the dependent claims.

The invention is based on the idea of keeping the sampling member off the test element in a common space until use, where only the sampling member is actuated. Accordingly with regard to a device it is proposed that the test member is fixed in a retaining chamber of the container, and the sampling member is configured in the same retaining chamber such that it is separated at a distance from the test member in an initial state and is in contact with the test member to transfer body fluid in a transfer state after the skin-piercing. In this manner it is possible to avoid a contamination of the sampling member especially with loose chemistry particles of the test element before the skin contact. Further, the fixed connection of the test member in the container facilitates manufacture of the disposable and allows a simplified sample transfer, while ensuring a safe disposal of the used and potentially bio-hazardous material. Accordingly, the sampling member is actuated to carry out a piercing movement outwards and backwards into the container, and the test member is arranged stationary inside the container.

In order to further improve the inventive concept, a preferred embodiment stipulates that the sampling member is separated from the test member by means of a spacer which can be put out of function during the skin-piercing. In order to provide a free space between the sampling member and the test member in the initial state, the spacer extents over a distance in direction of a piercing movement of the sampling member.

Advantageously, the spacer is removable by an actuator configured to actuate piercing movement of the sampling member. In this respect it is beneficial if the spacer is formed, preferably with a predetermined breaking point, at a wall of the container defining the retaining chamber, and is cut off the wall to achieve the transfer phase. Alternatively, it is possible that the spacer is connected to the sampling member, and a stopper of the container is removable such the spacer is inoperable in the transfer state.

Another improvement is achieved in that the sampling member is urged against the test member by means of a resilient element, thus ensuring an automatic sample transfer. In this configuration, the sampling member is deflected against a restoring force of the resilient element during the skin-piercing. This can be realized by a sampling member that has a flexible bending link forming the resilient element. In addition it is advantageous when the sampling member is formed as a hooked needle, and a spring-arm of the hooked needle is pre-tensioned against the test member.

For the application it is also advantageous the sampling member has a tip to pierce the skin and a capillary structure to uptake body fluid, wherein the capillary structure is in fluidic connection with the test element during the transfer phase. A preferred embodiment provides that the sampling member comprises a pointed piercing arm, and a laterally open channel is extending along the piercing arm preferably having a bent section that can be contacted with the test member.

In order to precisely define the actuation, the sampling member may comprise an engagement slot to receive an actuator rod for driving a piercing movement.

For the application it is also advantageous when the test member is formed as a reagent pad having a reactive layer which is sensitive to an analyte in the body fluid, and the flat-material pad is attached to a wall of the container defining the retaining chamber. In this configuration it is possible that the test member is configured to be mounted in the container as a unit separate from the sampling member.

Another improvement is achieved in that the container is sealed against the environment by a sealing foil, and at least a piercing part of the sampling member is penetrated through the sealing foil to allow the skin-piercing. The container can include a wall structure which defines the retaining chamber and comprises a desiccant material to keep the test chemistry dry.

A further advantage in usability is achieved when the container is formed as a magazine to contain a plurality of sampling members and test members preferably arranged in pairs in respective retaining chambers. To achieve a compact design, it is advantageous when the container comprises a disk-like configuration and contains a plurality of sampling members formed as a lancet wheel, and when the sampling members are actuated through a side face of the disk.

It is advantageous for a mass production when the lancet wheel is formed in one piece preferably by etching or cutting a metallic sheet material. It is further beneficial when the container includes a hub to engage a rotary drive for successive rotary positioning of the sampling members with respect to a sampling port of the apparatus constructed to receive the body part.

The invention can be used for analyzing body fluid especially for blood sugar tests in which a container is inserted into a measuring apparatus, a sampling member provided in the container is actuated to pierce skin of a body part and sample body fluid, and body fluid obtained by the skin-piercing is transferred to a test member, wherein the test member is stationary maintained in a retaining chamber of the container, and the sampling member is holded separated at a distance from the test member in an initial state and is brought in contact with the test member to transfer body fluid in a transfer state after the skin-piercing.

The invention is further elucidated in the following on the basis of the embodiment example shown schematically in the drawing figures.
- Fig. 1: shows a hand held measuring apparatus for blood glucose tests with an inserted disk-like cartridge;
- Fig. 2: shows the cartridge in a sectional diagrammatic view;
- Fig. 3: shows a chamber of the cartridge including a sampling member and a test member in a radial section;
- Fig. 4 to 6: show different states of the blood sampling and transfer in a view according to Fig. 3.

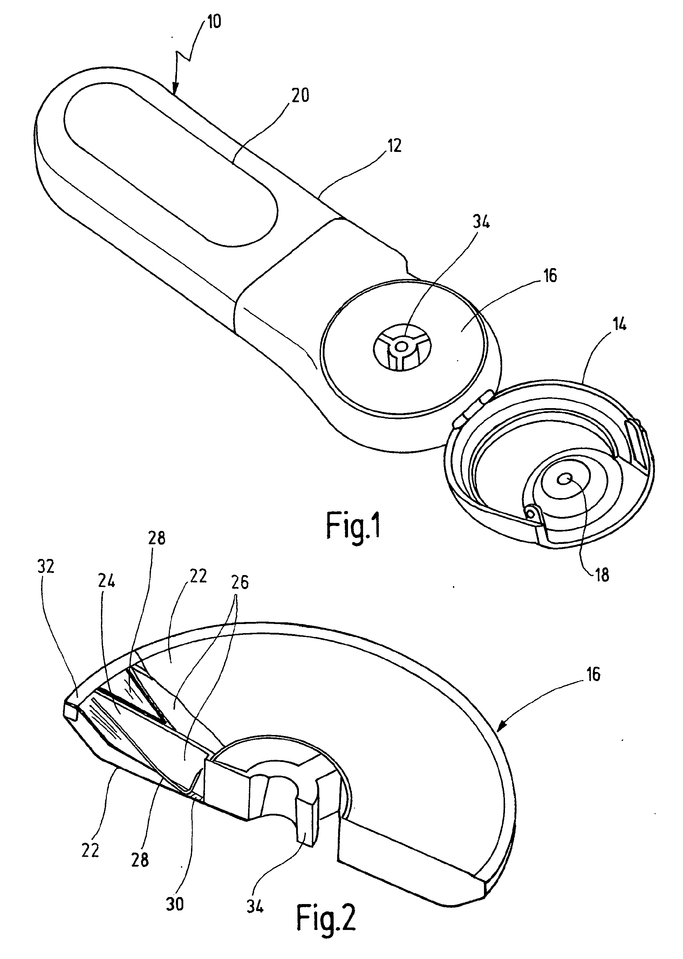

The apparatus 10 shown in Fig. 1 allows self-withdrawal of a blood sample by a user for analytical purposes and in particular for blood sugar monitoring. It comprises a housing 12 with a lid 14 to allow the change of a disposable test container or cartridge 16. In the closed state, a body part and in particular a fingertip can be pressed against a port 18 of the lid 14 in order to take a blood sample by skin-piercing. The apparatus 10 further comprises drive and processing units for an automatic measurement operation (not shown). The measurement result can be indicated on a display 20.

As shown in Fig. 2, the cartridge 16 comprises a disk-like configuration where the side faces are sealed against the environment by foils 22. The inner space between the foils is divided in sectors by a wall structure 24. Thereby, a magazine with a plurality of retaining chambers 26 is defined to accommodate in each chamber a sampling member 28 and a test member 30 in pair-wise configuration. The wall structure 24 can be formed as a single frame by injection molding with a desiccant material co-molded to keep the chambers 26 free from moisture. It is further conceivable to photo-etch or cut and then pre-form the plurality of sampling members 28 as a one piece lancet wheel 32 that can be inserted into the frame 26. In order to successively position the sampling members 28 with respect to the port 18, the frame 26 includes a hub 34 for engaging a rotary drive of the apparatus 10.

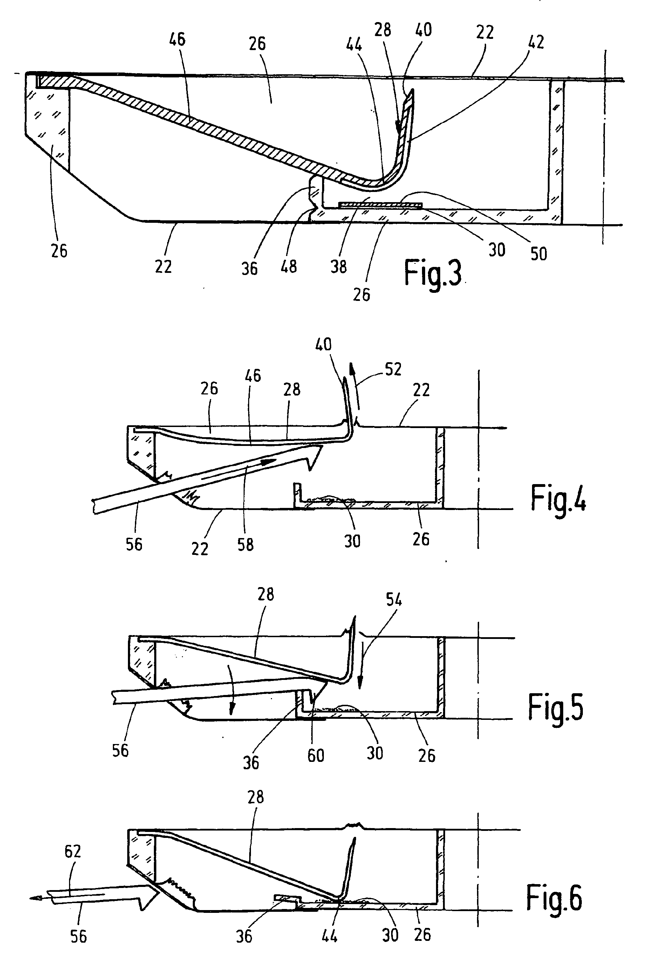

Fig. 3 shows an initial configuration of a retaining chamber 26 with the sampling member 28 being hold at a distance with respect to the test member 30 by means of a spacer 36. Due to the free space 38 kept by the spacer 36, the sampling member 28 is hold off the chemistry film of the test member 30 until the test is performed.

The sampling member 28 is formed as a hooked needle, which is accessible for actuation and allows piercing by break-through of the foils 22. For collecting a small amount of blood from a body part, the sampling member 28 comprises a pointed tip 40 and a capillary channel 42. The channel 42 is laterally open along his length and extents into a curved section 44 facing the test member 30. The sampling member 28 is linked to the periphery of the frame 26 via a flexible spring-arm 46. The spring-arm 46 is under pretension, such that the section 44 including the proximal part of the channel 42 is urged against the removable spacer 36 and the test member 30, respectively.

In order to allow a blood transfer from the channel 42 onto the test member 30, the spacer 36 can be removed during the skin-piercing procedure. This is facilitated by means of a breaking point 48 at the basis of the spacer 36. Alternatively, it is possible that the spacer is connected to the sampling member and abuts a removable counterpart, e.g. the sealing foil 22.

The test member 30 is formed as a reagent pad having a reactive layer 50. The reagent pad can be mounted as a unitary ring which is fixed to the bottom of the frame 26. The reactive layer 50 comprises a dried enzyme chemistry which is sensitive blood glucose, such that a colour change occurs which can be optically detected through the transparent frame 26. The detection of blood glucose in particular by means of contact-free optical methods is known in the prior art and is therefore not elucidated in more detail here.

Figs. 4 to 6 illustrate the sampling and transfer procedure, where the sampling member 28 is actuated to carry out a piercing movement outwards and backwards (arrows 52, 54) into the respective retaining chamber 26 of the cartridge 16, while the test member is kept stationary.

In the forward movement 52, the sampling member 28 is deflected against the restoring force of the spring-arm 46 by means of an actuator rod 56. The rod 56 is driven in direction of arrow 58 by a drive unit of the apparatus, thereby penetrating the bottom foil 22 and sliding along the spring-arm 46. It is also conceivable that the rod 56 is provided with an engaging piece for a form-locking engagement with the sampling member (not shown). While the actuator rod 56 is accessing the spring-arm 46, the needle tip 40 is breaking through the upper foil 22 and pierces the skin of the fingertip applied on the port 18. During the skin-piercing, blood and/or interstitial liquid is received in the channel 42 preferably by capillary action.

During the following resilient retraction 54 of the sampling member, the actuator rod 56 engages the spacer 36 with a hook piece 60 (Fig. 5). Subsequently, the rod 56 is pulled back in direction of arrow 62, whereby the spacer 36 is torn off the frame 26 (Fig. 6). This allows contact of the curved section 44 of the sampling member 28 with the test element 30, thereby initiating a transfer phase in which the sampled liquid is automatically transferred to the test element for an immediate analysis.

## Claims

1. A disposable device for analyzing body fluid especially for blood sugar tests comprising a container (16) which can be inserted into a measuring apparatus (10), a sampling member (28) provided in the container to pierce skin of a body part and sample body fluid, and a test member (30) for receiving body fluid obtained by the skin-piercing, wherein the test member is fixed in a retaining chamber (26) of the container, **characterized in that** the sampling member is configured in the same retaining chamber such that
- it is separate at a distance from the test member to provide a free space (38) between the sampling member and the test member in an initial state and
- it is in contact with the test member to transfer body fluid in a transfer state after the skin-piercing when the sampling member is in the retaining chamber.

2. The device according to claim 1, wherein the sampling member is actuated to carry out a piercing movement outwards and backwards into the container, and the test member is arranged stationary inside the container.

3. The device according to claim 1 or 2, wherein the sampling member is separated from the test member by means of a spacer (36) which can be put out of function during the skin-piercing.

4. The device according to claim 3, wherein the spacer extents over a distance in direction of a piercing movement of the sampling member in order to provide a free space between the sampling member and the test member.

5. The device according to claim 3 or 4, wherein the spacer is removable by an actuator (56) configured to actuate piercing movement of the sampling member.

6. The device according to any one of claims 3 to 5, wherein the spacer is formed, preferably with a predetermined breaking point, at a wall (24) of the container defining the retaining chamber, and that the spacer is cut off the wall to achieve the transfer phase.

7. The device according to any one of claims 1 to 6, wherein the sampling member is urged against the test member by means of a resilient element (46).

8. The device according to any one of claims 1 to 7, wherein the sampling member is formed as a hooked needle, and a spring-arm of the hooked needle is pre-tensioned against the test member.

9. The device according to any one of claims 1 to 8, in which the sampling member has a tip (40) to pierce the skin and a capillary structure (42) to uptake body fluid, wherein the capillary structure is in fluidic connection with the test element during the transfer phase.

10. The device according to any one of claims 1 to 9, wherein the test member is formed as a reagent pad having a reactive layer (50) which is sensitive to an analyte in the body fluid, and that the flat-material pad is attached to a wall of the container defining the retaining chamber.

11. The device according to any one of claims 1 to 10, wherein the test member is configured to be mounted in the container as a unit separate from the sampling member.

12. The device according to any one of claims 1 to 11, wherein the container is formed as a magazine to contain a plurality of sampling members and test members preferably arranged in pairs in respective retaining chambers.

13. The device according to any one of claims 1 to 12, wherein the container comprises a disk-like configuration and contains a plurality of sampling members formed as a lancet wheel (32), and the sampling members are actuated through a side face of the disk.

14. The device according to any one of claims 1 to 13, wherein the lancet wheel is formed in one piece preferably by etching or cutting a metallic sheet material.

15. The device according to any one of claims 1 to 14, wherein the container includes a hub to engage a rotary drive for successive rotary positioning of the sampling members with respect to a sampling port of the apparatus constructed to receive the body part.

## Patentansprüche

1. Einweg-Vorrichtung zur Analyse von Körperflüssigkeit insbesondere für Blutzucker-Tests mit einem in einen Messapparat (10) einsetzbaren Behälter (16), einem in dem Behälter bereitgestellten Sammelelement (28) zum Einstechen in die Haut eines Körperteils und zum Sammeln von Körperflüssigkeit, und einem Testelement (30) zum Aufnehmen von durch den Hauteinstich erhaltener Körperflüssigkeit, wobei das Testelement in einer Haltekammer (26) des Behälters fixiert ist, **dadurch gekennzeichnet, dass** das Sammelelement in der gleichen Haltekammer so konfiguriert ist, dass
- es in einem Ausgangszustand in einem Abstand von dem Testelement getrennt ist, um einen Freiraum (38) zwischen dem Sammelelement und dem Testelement zu schaffen, und
- es in einem Transferzustand nach dem Hauteinstich zum Übertragen von Körperflüssigkeit in Kontakt mit dem Testelement steht, wobei sich das Sammelelement in der Haltekammer befindet.

2. Vorrichtung nach Anspruch 1, wobei das Sammelelement zur Durchführung einer Stechbewegung nach außen und zurück in den Behälter betätigt wird, und das Testelement stationär in dem Behälter angeordnet ist.

3. Vorrichtung nach Anspruch 1 oder 2, wobei das Sammelelement von dem Testelement durch einen Abstandshalter (36) getrennt ist, welcher während des Hauteinstichs außer Funktion gebracht werden kann.

4. Vorrichtung nach Anspruch 3, wobei sich der Abstandhalter über einen Abstand in Richtung einer Stechbewegung des Sammelelements erstreckt, um einen Freiraum zwischen den Sammelelement und dem Testelement zu schaffen.

5. Vorrichtung nach Anspruch 3 oder 4, wobei der Abstandhalter durch einen zur Auslösung einer Stechbewegung des Sammelelements ausgelegten Aktuator (56) entfernbar ist.

6. Vorrichtung nach einem der Ansprüche 3 bis 5, wobei der Abstandhalter vorzugsweise mit einer Sollbruchstelle an einer die Haltekammer begrenzenden Wand (24) des Behälters gebildet ist, und wobei der Abstandshalter von der Wand abgeschnitten wird, um die Transferphase zu erlangen.

7. Vorrichtung nach einem der Ansprüche 1 bis 6, wobei das Sammelelement mittels eines elastischen Elements (46) gegen das Testelement gedrängt wird.

8. Vorrichtung nach einem der Ansprüche 1 bis 7, wobei das Sammelelement als Hakennadel ausgebildet ist, und ein Federarm der Hakennadel gegen das Testelement vorgespannt ist.

9. Vorrichtung nach einem der Ansprüche 1 bis 8, worin das Sammelelement eine Spitze (40) zum Einstechen in die Haut und eine Kapillarstruktur (42) zum Aufnehmen von Körperflüssigkeit aufweist, wobei die Kapillarstruktur während der Transferphase in fluidischer Verbindung mit dem Testelement steht.

10. Vorrichtung nach einem der Ansprüche 1 bis 9, wobei das Testelement als Reagenzfeld ausgebildet ist, das eine auf einen Analyten in der Körperflüssigkeit ansprechende reaktive Schicht (50) aufweist, und wobei das Flachmaterialfeld an einer die Haltekammer begrenzenden Wand des Behälters angebracht ist.

11. Vorrichtung nach einem der Ansprüche 1 bis 10, wobei das Testelement dazu ausgelegt ist, als von dem Sammelelement getrennte Einheit in dem Behälter angeordnet zu werden.

12. Vorrichtung nach einem der Ansprüche 1 bis 11, wobei der Behälter als Magazin zur Aufnahme einer Vielzahl von Sammelelementen und Testelementen vorzugsweise in paarweiser Anordnung in jeweiligen Haltekammern ausgebildet ist.

13. Vorrichtung nach einem der Ansprüche 1 bis 12, wobei der Behälter eine scheibenförmige Gestalt aufweist und eine Vielzahl von Sammelelementen als Lanzettenrad (32) enthält, und wobei die Sammelelemente durch eine Seitenfläche der Scheibe hindurch betätigt werden.

14. Vorrichtung nach einem der Ansprüche 1 bis 13, wobei das Lanzettenrad vorzugsweise durch Ätzen oder Schneiden eines metallischen Flachmaterials einstückig ausgebildet ist.

15. Vorrichtung nach einem der Ansprüche 1 bis 14, wobei der Behälter eine Nabe für den Eingriff eines Drehantriebs für aufeinanderfolgende Drehpositionierungen der Sammelelemente in Bezug auf einen zur Aufnahme des Körperteils ausgebildeten Sammelzugang des Apparats umfasst.

## Revendications

1. Dispositif jetable pour analyser un fluide corporel, spécialement pour des tests de glycémie, comprenant un récipient (16) qui peut être inséré dans un appareil de mesure (10), un élément de prélèvement d'échantillon (28) prévu dans le récipient pour percer la peau d'une partie corporelle et prélever un échantillon de fluide corporel, et un élément de test (30) pour recevoir le fluide corporel obtenu par le perçage de la peau, dans lequel l'élément de test est fixé dans une chambre de maintien (26) du récipient, **caractérisé en ce que** l'élément de prélèvement d'échantillon est configuré dans la même chambre de maintien de telle manière que
- il est séparé d'une distance de l'élément de test telle que pour prévoir un espace libre (38) entre l'élément de prélèvement d'échantillon et l'élément de test dans un état initial et
- il est en contact avec l'élément de test pour transférer le fluide corporel dans un état de transfert après le perçage de la peau lorsque l'élément de prélèvement d'échantillon est dans la chambre de maintien.

2. Dispositif selon la revendication 1, dans lequel l'élément de prélèvement d'échantillon est actionné de manière à exécuter un mouvement de perçage vers l'extérieur et vers l'arrière dans le récipient, et l'élément de test est agencé de façon stationnaire à l'intérieur du récipient.

3. Dispositif selon la revendication 1 ou 2, dans lequel l'élément de prélèvement d'échantillon est séparé de l'élément de test au moyen d'un espaceur (36) qui peut être mis hors service pendant le perçage de la peau.

4. Dispositif selon la revendication 3, dans lequel l'espaceur s'étend sur une distance dans le sens d'un mouvement de perçage de l'élément de prélèvement d'échantillon afin de créer un espace libre entre l'élément de prélèvement d'échantillon et l'élément de test.

5. Dispositif selon la revendication 3 ou 4, dans lequel l'espaceur peut être enlevé par un actionneur (56) configuré pour actionner un mouvement de perçage de l'élément de prélèvement d'échantillon.

6. Dispositif selon l'une quelconque des revendications 3 à 5, dans lequel l'espaceur est formé, préférablement avec un point de rupture prédéterminé, au niveau d'une paroi (24) du récipient définissant la chambre de maintien, et l'espaceur est découpé de la paroi pour réaliser la phase de transfert.

7. Dispositif selon l'une quelconque des revendications 1 à 6, dans lequel l'élément de prélèvement d'échantillon est poussé contre l'élément de test au moyen d'un élément résilient (46).

8. Dispositif selon l'une quelconque des revendications 1 à 7, dans lequel l'élément de prélèvement d'échantillon est formé comme une aiguille en crochet, et un bras de ressort de l'aiguille en crochet est pré-tendu contre l'élément de test.

9. Dispositif selon l'une quelconque des revendications 1 à 8, dans lequel l'élément de prélèvement d'échantillon a une pointe (40) pour percer la peau et une structure capillaire (42) pour récupérer un fluide corporel, dans lequel la structure capillaire est en connexion fluidique avec l'élément de test pendant la phase de transfert.

10. Dispositif selon l'une quelconque des revendications 1 à 9, dans lequel l'élément de test est formé comme un tampon de réactif ayant une couche réactive (50) qui est sensible à un analyte dans le fluide corporel, et le tampon d'un matériau plat est fixé à une paroi du récipient définissant la chambre de maintien.

11. Dispositif selon l'une quelconque des revendications 1 à 10, dans lequel l'élément de test est configuré pour être monté dans le récipient comme une unité séparée de l'élément de prélèvement d'échantillon.

12. Dispositif selon l'une quelconque des revendications 1 à 11, dans lequel le récipient est formé comme un magasin pour contenir une pluralité d'éléments de prélèvement d'échantillon et d'éléments de test préférablement agencés par paires dans des chambres de maintien respectives.

13. Dispositif selon l'une quelconque des revendications 1 à 12, dans lequel le récipient comprend une configuration de type disque et contient une pluralité d'éléments de prélèvement d'échantillon formée comme un barillet de lancettes (32), et les éléments de prélèvement d'échantillon sont actionnés à travers une face latérale du disque.

14. Dispositif selon l'une quelconque des revendications 1 à 13, dans lequel le barillet de lancettes est formé d'une seule pièce préférablement par gravure ou découpe d'un matériau de tôle métallique.

15. Dispositif selon l'une quelconque des revendications 1 à 14, dans lequel le récipient inclut un moyeu pour un engagement avec un entraînement rotatif pour un positionnement rotatif successif des éléments de prélèvement d'échantillon par rapport à un orifice de prélèvement d'échantillon de l'appareil construit pour recevoir la partie corporelle.
